(19) Europäisches Patentamt
European Patent Office
Office européen des brevets

(11) Veröffentlichungsnummer : **0 378 204 B1**

(12) **EUROPÄISCHE PATENTSCHRIFT**

(45) Veröffentlichungstag der Patentschrift : **24.05.95 Patentblatt 95/21**

(51) Int. Cl.$^6$ : **G01N 33/53,** C07D 339/04, G01N 33/74, G01N 33/78, G01N 33/82

(21) Anmeldenummer : **90100463.0**

(22) Anmeldetag : **10.01.90**

(54) **Verfahren zur Ablösung eines Analyten von seinem Bindeprotein.**

(30) Priorität : **11.01.89 DE 3900649**

(43) Veröffentlichungstag der Anmeldung : **18.07.90 Patentblatt 90/29**

(45) Bekanntmachung des Hinweises auf die Patenterteilung : **24.05.95 Patentblatt 95/21**

(84) Benannte Vertragsstaaten : **AT BE CH DE DK ES FR GB GR IT LI LU NL SE**

(56) Entgegenhaltungen :
**WO-A-82/03461**
**WO-A-89/05975**
**GB-A- 2 084 320**

(73) Patentinhaber : **BOEHRINGER MANNHEIM GMBH**
**D-68298 Mannheim (DE)**

(72) Erfinder : **Hoyle, Nicholas R., Dr.**
**Bräuhausstrasse 25**
**D-8132 Tutzing (DE)**

(74) Vertreter : **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**D-81635 München (DE)**

EP 0 378 204 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Ablösung eines an ein Bindeprotein gebundenen Analyten von diesem Bindeprotein durch Abbau des Bindeproteins durch ein SH-Gruppen spaltendes Thiol.

Viele interessante Analyte, z.B. Vitamine oder Steroide, sind spezifisch oder unspezifisch an Bindeproteine, z.B. Serumproteine oder andere natürliche Bindestoffe, gebunden. Bevor sie bestimmt werden können, müssen die Analyte von ihren Bindeproteinen oder den anderen Bindestoffen gelöst werden. In vielen Fällen kann dies durch Verwendung des Kompetitionsprinzipes erreicht werden, z.B. durch Addition eines Überschusses von kreuzreaktiven Analogen des Analyten, oder durch Einhaltung milder, dissoziativer Bedingungen, wie z.B. durch Zusatz von 8-Anilino-1-naphthalin-sulfonsäure (ANS), um $T_4$ von Thyroxin-bindendem Globulin (TBG) zu lösen. In einigen Fällen ist die Bindung aber so fest, daß stärker zerstörende Methoden nötig sind, wie z.B. zur Ablösung von Vitamin B12.

Die derzeit gebräuchlichen Methoden zur Bestimmung von Analyten, wie z.B. von Vitamin B12 (Cyanocobalamin) in stark verdünnten wäßrigen Lösungen (wie z.B. dem Blutserum) basieren auf Verfahren unter Verwendung radioaktiver Markierungsstoffe, in denen Intrinsic Factor (IF) als bindendes Reagenz verwendet wird. Die gebräuchlichen Techniken arbeiten unter Verwendung von $^{57}$Co.B12 als Marker unter Zugrundelegung eines kompetitiven Prinzips, bei dem freie und markierte Analyte um die Bindung mit dem IF kompetieren. Die Trennung der gebundenen und freien Proben (bound/free-Trennung) erfolgt dann aufgrund von Methoden wie z.B. unter Verwendung von Aktivkohle, festphasengebundenem IF oder magnetischer Trennung, bei der IF an paramagnetische Partikel gebunden ist.

Die derzeit gebräuchlichen Methoden zur Ablösung des Analyten, z.B. von Vitamin B12, vom Serum-Bindeprotein (Probenvorbereitung) basieren üblicherweise auf der Zerstörung des Bindeproteins im alkalischen Bereich (pH 13,5) unter der Einwirkung des SH-Gruppen spaltenden Thiols Dithiothreitol (DTT) (Inkubation der Serumprobe mittels DTT im alkalischen Bereich), oder aber durch 30- bis 60minütiges Kochen und nachfolgende Zentrifugation. Durch Zugabe von organischen Stoffen, wie z.B. Aceton, Alkohol, oder durch Zugabe von kompetitiven, kreuzreagierenden Spezien, z.B. von Cobinamid, kann diese Zerstörung verstärkt werden. Alle üblichen Testmethoden enthalten außerdem Kaliumcyanid, um die Extraktionsfähigkeit des Vitamin B12 zu steigern, und um die Cobalamine in eine gleichförmige, stabile und nachweisbare Form, nämlich das Cyanocobalamin, zu überführen.

Nachteile dieser beiden derzeit gebräuchlichen Techniken der Probenvorbereitung sind insbesondere bei Verwendung von DTT die geringe Ablösungseffektivität (nur ca. 60 bis 80%), begrenzte Stabilität und unangenehmer Geruch. Die Hitzeablösung ist im Routinelabor durch den erforderlichen technischen Aufwand und den Zeitaufwand für das Kochen und die Zentrifugation unpraktikabel.

Aufgabe der vorliegenden Erfindung war es deshalb, ein Verfahren zur Probenvorbereitung bereitzustellen, mit dem die vorstehend genannten Mängel der bisher gebräuchlichen Probenvorbereitungsmethoden behoben werden können. Diese Aufgabe wird mit der vorliegenden Erfindung gelöst.

Gegenstand der Erfindung ist ein Verfahren zur Ablösung eines an ein Bindeprotein gebundenen Analyten durch Abbau des Bindeproteins durch ein SH-Gruppen spaltendes Thiol im alkalischen Bereich, das dadurch gekennzeichnet ist, daß man als SH-Gruppen spaltendes Thiol eine 1,2-Dithiolan-3-carbonsäure der Formel (I)

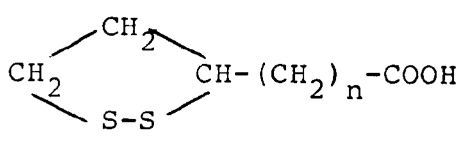

(I)

verwendet, worin n eine ganze Zahl von 1 bis 8, vorzugsweise 3 bis 5 bedeutet.

Als Säure der Formel I wird bevorzugt Liponsäure verwendet. Als alkalisches Medium wird zweckmäßig eine Alkalihydroxid-Lösung, insbesondere eine NaOH- oder KOH-Lösung eingesetzt. Die Alkalikonzentration liegt dabei vorzugsweise im Bereich von 0,05 bis 1 mol/l. Die bevorzugte Konzentration der Carbonsäure gemäß Formel I beträgt 1 bis 20 mg/ml, bevorzugt 4 bis 10 mg/ml (berechnet für Liponsäure mit n = 4).

Das erfindungsgemäße Verfahren eignet sich grundsätzlich zur Ablösung aller Analyten, die an spezifische Bindeproteine gebunden vorliegen, insbesondere zur Ablösung von Vitaminen, Thyroidhormonen oder Steroiden. Für die Freisetzung von Vitamin B12 als Analyt erwies sich das Verfahren der Erfindung als besonders geeignet.

Gemäß einer bevorzugten Ausführungsform der Erfindung wird das Verfahren in Gegenwart von KCN durchgeführt. Die Konzentration an KCN liegt zweckmäßig zwischen 1 und 10 mg/ml, jedoch können auch größere oder kleinere Konzentrationen schon zu einer weiteren Verbesserung der Ablösung führen.

In der Formel (I) bedeutet n vorzugsweise 3 bis 5, und insbesondere 4 (Liponsäure).

Als alkalisches Medium wird vorzugsweise ein Alkalihydroxid verwendet, und insbesondere NaOH oder KOH. Die Alkalikonzentration liegt dabei zweckmäßigerweise im Bereich von 0,05 bis 1 mol/l, vorzugsweise bei 0,2 bis 0,7 mol/l. Der pH-Wert des Mediums liegt dabei bei mindestens 10, im allgemeinen zwischen 10 und 14, und vorzugsweise zwischen 13 und 14, und ist besonders bevorzugt 13,6.

Die 1,2-Dithiolan-3-carbonsäure der Formel (I) wird (für Liponsäure mit n=4 berechnet) vorzugsweise in einem Bereich von 1 bis 20 mg/ml, insbesondere im Bereich von 4 bis 10 mg/ml, und in erster Linie zwischen 5 und 8 mg/ml eingesetzt. Zur Bestimmung von Vitamin B12 wird vorzugsweise in Gegenwart eines Cyanids, z.B. Kaliumcyanid, gearbeitet. Das Cyanid wird zweckmäßigerweise in einer Menge von 0,5 bis 5 mg/ml, und insbesondere von 1 mg/ml eingesetzt.

Das erfindungsgemäße Verfahren zur Probenvorbereitung eignet sich sowohl für manuelle Bestimmungsverfahren als auch für Verfahren unter Verwendung automatischer Analysengeräte.

Die Vorteile des erfindungsgemäßen Verfahrens sind insbesondere:

Die Zerstörung des Bindeproteins erfolgt sehr rasch, und in der Regel in weniger als 15 Minuten; das Verfahren ist sehr effizient, mit einer Ablösung von 80 bis 95%; die Reagenzlösung zur Probenvorbereitung ist sehr stabil (Lagerfähigkeit bei Raumtemperatur mehr als 8 Wochen); unter Verwendung des erfindungsgemäßen Ablösereagenzes tritt kein unangenehmer Geruch auf, und das Reagenz ist völlig ungiftig; das Reagenz ist universell anwendbar; aufgrund des Bestrebens der Liponsäure, an die Proteine zu binden, während sie diese zerstört, ist die Beeinflussung des Tests sehr gering, und die Möglichkeit der Rückbildung der Protein- und Analyt-Komplexe beim Neutralstellen des pH-Wertes wird stark eingeschränkt.

Das erfindungsgemäße Verfahren zur Ablösung eines an ein Bindeprotein gebundenen Analyten von diesem Bindeprotein eignet sich zur Probenvorbereitung für eine Vielzahl von Bestimmungsmethoden. Vorzugsweise eignet sich das erfindungsgemäße Verfahren für Analyte wie z.B. Vitamine, Thyroidhormone und Steroide, z.B. zur Ablösung von Testosteron von Testosteron-bindenden Globulinen; $T_3$, $T_4$ von Thyroxin-bindenden Globulinen (TBG), aber auch für andere Systeme, bei denen der Analyt an ein Protein gebunden ist, wie z.B. an eine Cellmatrix oder Bindeprotein gebundene Tumormarker, und insbesondere zur Probenvorbereitung in Vitamin B12-Bestimmungsverfahren.

Verfahren zur Bestimmung für derartige Analyte sind beispielsweise beschrieben in K. Lübke, Immunologische Teste für niedermolekulare Wirkstoffe, Georg Thieme Verlag, Stuttgart (1978), Clin.Chim. Acta 22 (1968) 51-69; B. Rothfeld ed. Nuclear Medicine Lippincott, Philadelphia 69-84, 1974 sowie insbesondere für Testosteron in J. Endocr. 100 (1984) 367-376, J. Steroid. Biochem. 19 (1983) 1605-1610 sowie J.Steroid.Biochem. 22 (1985) 169-175 und DE-A 3545252. Verfahren zur Bestimmung von T3 und T4 sind beispielsweise beschrieben in Biochem.Biophys.Res.Comm. 46 (1972) 2107-2113. Verfahren zur Bestimmung von Vitamin B12 sind beispielsweise beschrieben in Clinical Biochemistry 18 (1985) 261-266, J.Clin.Path. 20 (1967) 683-686, Brit.Haemat. 22 (1972) 21-31, Biochem.Biophys.Res.Comm. 46 (1972) 2107-2113, sowie in der DE-A 3900650 (Titel: "Vitamin B12-Bestimmung") der gleichen Anmelderin mit dem gleichen Anmeldetag.

Ein weiterer Gegenstand der vorliegenden Erfindung ist auch ein Reagenz zur Probenvorbereitung für die Bestimmung von Vitamin B12 im Blutserum, enthaltend 1 bis 20 mg/ml Liponsäure und 1 bis 10 mg/ml Alkalicyanid bei pH 10 bis 14.

Das Reagenz zur Probenvorbereitung wird zur Probe zugegeben und diese damit vorzugsweise 10 bis 20, insbesondere 15 Minuten bei Raumtemperatur inkubiert. Anschließend wird der pH-Wert auf Werte zwischen 7 und 9 abgesenkt. Vorzugsweise wird dies langsam, insbesondere in zwei Schritten, unter Verwendung geeigneter Pufferlösungen durchgeführt, da sonst Niederschläge auftreten können.

Die Probenvorbereitung erfolgt nach dem erfindungsgemässen Verfahren, insbesondere unter Verwendung von Liponsäure (Formel I, n=4). Zweckmäßigerweise arbeitet man in Gegenwart von KCN (vorzugsweise bei einer Konzentration von 1 bis 10 mg/ml, insbesondere von 1 bis 5 mg KCN/ml). Die Inkubation wird vorzugsweise 15 Minuten bei Raumtemperatur im alkalischen Bereich durchgeführt, und vor der Durchführung der Verfahrensstufe c) wird neutralisiert. Anstelle von KCN kann auch NaCN oder ein anderes leicht dissoziierendes Cyanid mit einem das Verfahren nicht beeinflussenden Kation verwendet werden.

Als kompetitives markiertes B12 (Marker) in Stufe d) kann z.B. $^{57}$Co.B12 verwendet werden. Um die mit radioaktiven Markern verbundenen Nachteile zu vermeiden, wird zweckmäßigerweise ein B12-Konjugat der Formel (II)

$$\text{B12-CO-NH}\left(\text{NH-R-CO-NH}\right)_x\text{N=GP} \qquad \text{(II)}$$

verwendet, worin B12 den aus Cyanocobalamin (Vitamin B12) durch Abspaltung einer $-CONH_2$-Gruppe gebildeten Rest und R eine Verknüpfungsgruppierung (Spacer) bedeutet, und x 0 oder 1 ist, und GP den Rest eines glykosylgruppenhaltigen Markerenzyms darstellt, das über einen Glykosylrest an die -NH-N=-Gruppierung gebunden ist. In der Formel (II) steht die -CONH-Gruppierung vorzugsweise in d-Stellung des B12-Restes, und in erster Linie werden B12-d-CO-NH-N=GP, und insbesondere B12-d-CO-NH-NH-CO-$CH_2$$\left( O\text{-}CH_2\text{-}CH_2 \right)_3$O-$CH_2$-CO-NH-N=GP, worin GP den Peroxidaserest (POD) bedeutet, verwendet.

Die B12-Konjugate der Formel (II) sind Gegenstand der DE-A 3900648 (Titel: Neue Cobalamin-Säurehydrazide und davon abgeleitete Cobalamin-Konjugate) der gleichen Anmelderin mit dem gleichen Anmeldetag. Sie lassen sich durch Kupplung (Kondensation) der Cobalamin-Säurehydrazide der Formel

$$B12\text{-}CO\text{-}NH\text{-}NH\left(R\text{-}CO\text{-}NH\text{-}NH\right)_x H$$

(worin B12, R und x die vorstehend genannte Bedeutung besitzen), die ebenfalls Gegenstand der vorstehend genannten gleichzeitig eingereichten DE-A 3900648 sind, mit den OH-Gruppen von Glykosylresten der Glykoproteine nach deren Oxidation und Ausbildung der Hydrazongruppierung -NH-N=CH-Glykoprotein unter an sich bekannten Bedingungen herstellen.

Als Markierungsanalyt wird insbesondere

$$B12\text{-}d\text{-}CO\text{-}NH\text{-}NH\text{-}CO\text{-}CH_2\left(O\text{-}CH_2\text{-}CH_2\right)_3 O\text{-}CH_2\text{-}CO\text{-}NH\text{-}N\text{=}POD$$

verwendet.

Die Bestimmung der POD kann auf eine an sich bekannte Weise erfolgen. Zweckmäßigerweise erfolgt die Bestimmung der POD über eine Anfärbung von POD mit dem Diammoniumsalz der 2,2′-Azinodi-(3-ethylbenzthiazolin-6-sulfonsäure) (ABTS) als Chromogen.

Die Vorbehandlung der Probe (Stufe b)) kann extern, oder auch im Rahmen eines automatischen Vitamin B12-Bestimmungsprogrammes, z.B. unter Verwendung der vorstehend genannten automatischen Analysengeräte, erfolgen. Die Menge an B12-Konjugat, das in Lösung, als Konzentrat, oder auch in lyophilisierter Form eingesetzt werden kann, ist insbesondere abhängig von der Art der Proben und dem darin zu erwartenden B12-Gehalt. Die optimale Menge wird zweckmäßigerweise für einen bestimmten, zu erwartenden B12-Gehalt vorher festgestellt.

Mit dem Verfahren zur Bestimmung von Vitamin B12 unter Verwendung der erfindungsgemäßen Probenvorbereitung, insbesondere unter gleichzeitiger Verwendung der vorstehend genannten Antikörper als Bindereagenz für Vitamin B12, wird ein Testsystem bereitgestellt, mit dem die Probleme, die bei der Verwendung der bisher üblichen Probenvorbereitung in Verbindung mit radioaktiv markiertem B12 auftreten, vermieden werden. Das erfindungsgemäße System ist nicht gesundheitsgefährlich, und die Reagenzien weisen eine wesentlich größere Haltbarkeit auf, als dies bei bisher üblichen Testsystemen der Fall ist. Durch die leichte Handhabung und Ungefährlichkeit der verwendeten Reagenzien und die leichte Durchführung des Tests ist das System insgesamt sehr anwenderfreundlich. Die verwendeten monoklonalen Antikörper werden durch Serumantikörper, die das bisher verwendete IF binden oder blockieren können, nicht beeinflußt. Die Verwendung der erfindungsgemäßen Ablösemethode (Probenvorbereitung) trägt zur Verbesserung der Testgenauigkeit und zur größeren Akzeptanz des Testes bei. Es wird eine bessere Reproduzierbarkeit und eine größere Effizienz der Vitamin B12-Ablösung erreicht.

Die nachfolgenden Beispiele und die Abbildung sollen die Erfindung näher erläutern, ohne sie darauf zu beschränken. Unter Raumtemperatur (RT) wird eine Temperatur von 25°C ± 2°C verstanden. Prozentangaben beziehen sich auf Gewichtsprozent.

Figur 1 zeigt Standardkurven für die Vitamin B12-Bestimmung nach Beispiel 3 ohne Zusatz (Kurve 1), mit Zusatz von 10 mg/ml Liponsäure (Kurve 2) und mit Zusatz von 10 mg/ml DTT (Kurve 3).

Beispiel 1

Probenvorbehandlung mit Liponsäure

250 µl Humanserum werden mit 125 µl Ablösereagenz (bestehend aus 8 mg/ml Liponsäure, 1 mg/ml Kaliumcyanid, gelöst in 0,5 mol/l NaOH) gemischt und 15 min bei Raumtemperatur inkubiert. Anschließend werden 125 µl 200 mmol/l Phosphatpuffer, pH 4,1 zugegeben.

Anstelle von 200 mmol/l Phosphatpuffer, pH 4,1 können auch 25 µl Phosphorsäure ($H_3PO_4$, 1,5 N) verwendet werden. Dies hat den Vorteil, daß dabei die Verdünnung der Probe verringert wird.

In einer weiteren Variante können 800 µl Phosphatpuffer (200 mmol/l, pH 7,2) zur Neutralisation zugegeben werden.

Beispiel 2

Probenvorbehandlung mit DTT (Vergleich)

250 µl Humanserum werden mit 125 µl DTT-Ablösereagenz (10 mg/ml DTT, 1 mg/ml Kaliumcyanid, gelöst

in 0,5 mol/l NaOH) gemischt und 15 min bei Raumtemperatur inkubiert. Anschließend werden 125 µl Phosphatpuffer (200 mmol/l, pH 4,1) zugegeben.

Beispiel 3

Bestimmung von Vitamin B12

a) Reagenzien:

Polystyrolröhrchen, beschichtet mit Thermo-RSA Streptavidin (hergestellt nach EP-A 0269092)
Reagenz 1
95 ng/ml biotinylierter MAK gegen Vitamin B12 (ECACC 88101302)
(Biotinylierung nach JACS 100 (1978) 3585-3590) 40 mmol/l Phosphatpuffer, pH 7,2
Reagenz 2

B12-d-CO-NH-NH-CO-CH2-(-O-CH2-CH2-)$_3$-O-CH2-CO-NH-N = POD)

(Aktivität ca. 60 mU/ml)
40 mmol/l Phosphatpuffer, pH 7,2
Reagenz 3
100 mmol/l Phosphat-Citratpuffer, pH 4,4
1,9 mmol/l ABTS® (2,2′-Azino-di[3-ethyl-benzthiazolinsulfonat])
3,2 mmol/l Natriumperborat

b) Durchführung der Bestimmung

Zur Durchführung der Bestimmung werden 200 µl nach Beispiel 1 oder Beispiel 2 vorbehandelte Probe mit 800 µl Reagenz 1 in ein Streptavidin-tube gegeben und 60 min bei Raumtemperatur inkubiert. Anschließend wird mit Waschlösung (250 mg/ml Natriumchlorid, 1 mg/100 ml Kupfersulfat) gewaschen und 1000 µl Reagenz 2 zugegeben und 30 min bei Raumtemperatur inkubiert. Es wird mit Waschlösung (250 mg/ml Natriumchlorid, 1 mg/100 ml Kupfersulfat) gewaschen und Reagenz 3 zugegeben, für 30 min bei Raumtemperatur inkubiert und die gebildete Farbe als Maß für den Vitamin B12-Gehalt bei 420 nm gemessen.

Tabelle 1 zeigt einen Vergleich der Ergebnisse für verschiedene Humanseren bei Verwendung von Liponsäure bzw. DTT als Ablösereagenzien im Test nach Beispiel 3

Tabelle 1

| B12 Konzentration (pg/ml) | |
|---|---|
| Liponsäure 10 mg/ml | DTT 10 mg/ml |
| 376 | 94 |
| 452 | 174 |
| 418 | 252 |
| 513 | 152 |
| 3939 | 458 |

Daraus ergibt sich, daß bei Verwendung von Liponsäure höhere Meßwerte und damit eine bessere Ablösung erreicht werden kann.

Fig. 1 zeigt bei Verwendung von Cyancobalamin-Standards (Cyanocobalamin in 40 mmol/l Phosphatpuffer, pH 7,2 mit 0,9 % Natriumchlorid, 0,9 % Crotein C und 0,1 % Kaliumcyanid den Einfluß von DTT (10 mg/ml) bzw. Liponsäure (10 mg/ml) auf die Eichkurve. Danach wird die Eichkurve von Liponsäure nur sehr gering beeinflußt.

Analoge Ergebnisse werden erhalten, wenn als MAK gegen B12 ein MAK aus der Zellinie ECACC 88101301 eingesetzt wird.

Beispiel 4

Testosteron-Bestimmung

Reagenzien:

Testpuffer:

35 ng/ml monoklonaler Antikörper gegen Testosteron (ECACC 85121701) 40 mmol/l Natriumphosphat, pH 6,8

Beladungslösung:

| | |
|---|---|
| 10 µg/ml | polyklonaler Schaf-Antikörper (IgG) gegen Maus Fc-gamma |
| 20 mmol/l | Natriumcarbonatpuffer, pH 9,6 |

Waschlösung:

| | |
|---|---|
| 250 mg/100 ml | Natriumchlorid |
| 1 mg/100 ml | Kupfersulfat |

Substratlösung:

| | |
|---|---|
| 1,9 mmol/l ABTS® | (2,2″Azino-di-[3-ethyl-benzthiazolinsulfonsäure(6)]-Diammoniumsalz |
| 100 mmol/l | Phosphat-Citratpuffer, pH 4,4 |
| 3,2 mmol/l | Natrium-Perborat |

Probe:

Humanserum vorbehandelt mit Liponsäure nach Beispiel 1 oder mit DTT nach Beispiel 2.

Zur Durchführung der Bestimmung wurden 1 ml Beladungslösung 30 Minuten bei Raumtemperatur in einem Luran-Röhrchen inkubiert. Anschließend wurde zweimal mit Waschlösung gewaschen. Es wurde 1 ml Testpuffer zugegeben, 30 Minuten bei Raumtemperatur inkubiert und zweimal mit Waschlösung gewaschen. Es wurden 100 µl Probe und 1 ml Testosteron 3-cmo-POD-Konjugat (hergestellt nach DE 3833149, 80 mU/ml) in 40 mmol/l Natriumphosphatpuffer, pH 6,8, mit 0,2 % Pluronic F68 zugegeben, 30 Minuten bei Raumtemperatur inkubiert und zweimal mit Waschlösung gewaschen. Danach wurde 1 ml Substratlösung zugegeben, 30 Minuten bei Raumtemperatur inkubiert und die Extinktion bei 405 nm gemessen.

## Patentansprüche

### Patentansprüche für folgende Vertragsstaaten : AT, BE, CH, LI, DE, DK, FR, GB, GR, IT, LU, NL, SE

1. Verfahren zur Ablösung eines an ein Bindeprotein gebundenen Analyten durch Abbau des Bindeproteins durch ein SH-Gruppen spaltendes Thiol im alkalischen Bereich,
   **dadurch gekennzeichnet,**
   daß man als SH-Gruppen spaltendes Thiol eine 1,2-Dithiolan-3-carbonsäure der Formel (I)

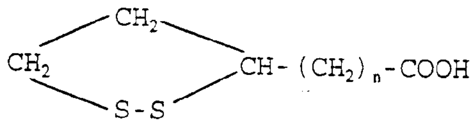

(I)

verwendet, worin n eine ganze Zahl von 1 bis 8 bedeutet.

2. Verfahren nach Anspruch 1, **dadurch gekennzeichnet,** daß in der Formel (I) n 3 bis 5 bedeutet.

3. Verfahren nach Anspruch 1 oder 2, **dadurch gekennzeichnet,** daß man als Säure der Formel (I) Liponsäure verwendet.

4. Verfahren nach einem der Ansprüche 1 bis 3, **dadurch gekennzeichnet**, daß die Konzentration der 1,2-Dithiolan-3-carbonsäure der Formel (I) (für Liponsäure mit n=4 berechnet) 1 bis 20 mg/ml beträgt.

5. Verfahrennach einem der Ansprüche 1 bis 4, **dadurch gekennzeichnet**, daß der freizusetzende Analyt ein vitamin, thyroidhormon oder Steroid ist.

6. Verwendung des Verfahrens nach einem der Ansprüche 1 bis 5 zur Probenvorbereitung für Analysen- und Untersuchungsverfahren zur Bestimmung des freigesetzten Analyten, insbesondere für Immunoassays.

7. Verwendung einer 1,2-Dithiolan-3-carbonsäure der Formel (I),

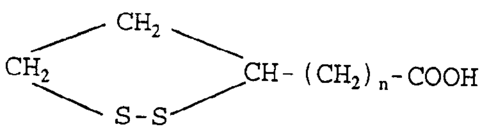

(I)

worin n eine ganze Zahl von 1 bis 8, vorzugsweise 3 bis 5 und insbesondere 4 bedeutet, zur Ablösung eines an ein Bindeprotein gebundenen Analyten als Probenvorbereitung zu seiner Bestimmung, insbesondere durch Immunoassay.

8. Reagenz zur Probenvorbereitung für die Bestimmung von Vitamin B12 im Blutserum, enthaltend
1 bis 20 mg/ml Liponsäure,
1 bis 10 mg/ml Alkalicyanid
bei pH 10 bis 14.

**Patentansprüche für folgenden Vertragsstaat : ES**

1. Verfahren zur Ablösung eines an ein Bindeprotein gebundenen Analyten durch Abbau des Bindeproteins durch ein SH-Gruppen spaltendes Thiol im alkalischen Bereich, **dadurch gekennzeichnet**, daß man als SE-Gruppen spaltendes Thiol eine 1,2-Dithiolan-3-carbonsäure der Formel (I)

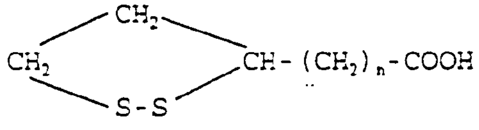

(I)

verwendet, worin n eine ganze Zahl von 1 bis 8 bedeutet.

2. Verfahren nach Anspruch 1,
**dadurch gekennzeichnet,**
daß in der Formel (I) n 3 bis 5 bedeutet.

3. Verfahren nach Anspruch 1 oder 2,
**dadurch gekennzeichnet,**
daß man als Säure der Formel (I) Liponsaüre verwendet.

4. Verfahren nach einem der Ansprüche 1 bis 3,
**dadurch gekennzeichnet,**
daß die Konzentration der 1,2-Dithiolan-3-carbonsäure der Formel (I) (für Liponsäure mit n=4 berechnet) 1 bis 20 mg/ml beträgt.

5. Verfahren nach einem der Ansprüche 1 bis 4,
**dadurch gekennzeichnet,**
daß der freizusetzende Analyt ein Vitamin, Thyroidhormon oder Steroid ist.

6. Verwendung des Verfahrens nach einem der Ansprüche 1 bis 5 zur Probenvorbereitung für Analysen- und Untersuchungsverfahren zur Bestimmung des freigesetzten Analyten, insbesondere für Immunoassays.

7. Verwendung einer 1,2-Dithiolan-3-carbonsäure der Formel (I),

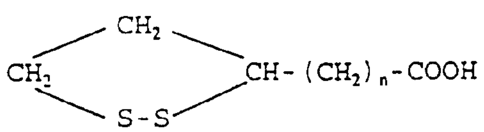

(I)

worin n eine ganze Zahl von 1 bis 8, vorzugsweise 3 bis 5 und insbesondere 4 bedeutet, zur Ablösung eines an ein Bindeprotein gebundenen Analyten als Probenvorbereitung zu seiner Bestimmung, insbesondere durch Immunoassay.

8. Verfahren zur Herstellung eines Reagenzes zur Probenvorbereitung für die Bestimmung von Vitamin B12 im Blutserum, dadurch gekennzeichnet, daß
1 bis 20 mg/ml Liponsäure,
1 bis 10 mg/ml Alkalicyanid
bei pH 10 bis 14, als aktive Inhaltsstoffe verwendet werden.

## Claims

**Claims for the following Contracting States : AT, BE, CH, LI, DE, DK, FR, GB, GR, IT, LU, NL, SE**

1. Process for the removal of an analyte bound to a binding protein by breakdown of the binding protein by a thiol cleaving SH groups in the alkaline range, characterised in that, as SH group-cleaving thiol, one uses a 1,2-dithiolan-3-carboxylic acid of the formula (I)

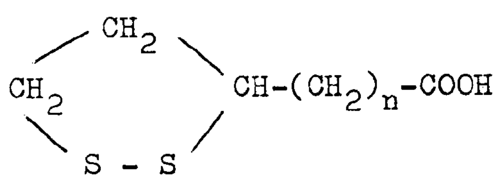

(I)

wherein n signifies a whole number of 1 to 8.

2. Process according to claim 1, characterised in that n signifies 3 to 5 in the formula (I).

3. Process according to claim 1 or 2, characterised in that one uses liponic acid as acid of the formula (I).

4. Process according to one of claims 1 to 3, characterised in that the concentration of the 1,2-dithiolan-3-carboxylic acid of the formula (I)(calculated for liponic acid with n = 4) amounts to 1 to 20 mg/ml.

5. Process according to one of claims 1 to 4, characterised in that the analyte to be liberated is a vitamin, thyroid hormone or steroid.

6. Use of the process according to one of claims 1 to 5 for the sample preparation for analysis or investigation processes for the determination of the liberated analyte, especially for immunoassays.

7. Use of a 1,2-dithiolan-3-carboxylic acid of the formula (I)

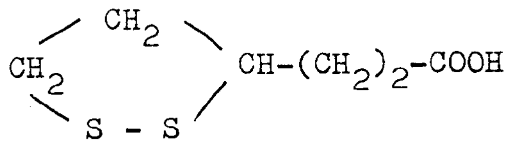

(I)

wherein n signifies a whole number of 1 to 8, preferably of 3 to 5 and especially of 4, for the removal of an analyte bound to a binding protein as sample preparation for its determination, especially by immunoassay.

8. Reagent for the sample preparation for the determination of vitamin B12 in blood serum, containing 1 to 20 mg/ml of liponic acid, 1 to 10 mg/ml of an alkali metal cyanide at pH 10 to 14.

**Claims for the following Contracting State : ES**

1. Process for the removal of an analyte bound to a binding protein by breakdown of the binding protein by a thiol cleaving SH groups in the alkaline range, characterised in that, as SH group-cleaving thiol, one uses a 1,2-dithiolan-3-carboxylic acid of the formula (I)

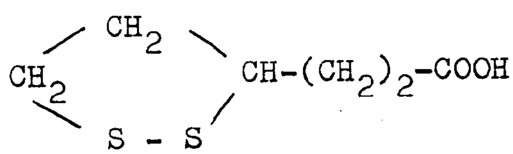

(I)

wherein n signifies a whole number of 1 to 8.

2. Process according to claim 1, characterised in that n signifies 3 to 5 in the formula (I).

3. Process according to claim 1 or 2, characterised in that one uses liponic acid as acid of the formula (I).

4. Process according to one of claims 1 to 3, characterised in that the concentration of the 1,2-dithiolan-3-carboxylic acid of the formula (I)(calculated for liponic acid with n = 4) amounts to 1 to 20 mg/ml.

5. Process according to one of claims 1 to 4, characterised in that the analyte to be liberated is a vitamin, thyroid hormone or steroid.

6. Use of the process according to one of claims 1 to 5 for the sample preparation for analysis or investigation processes for the determination of the liberated analyte, especially for immunoassays.

7. Use of a 1,2-dithiolan-3-carboxylic acid of the formula (I)

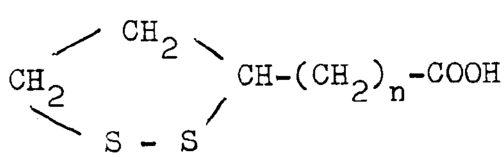

(I)

wherein n signifies a whole number of 1 to 8, preferably of 3 to 5 and especially of 4, for the removal of an analyte bound to a binding protein as sample preparation for its determination, especially by immunoassay.

**8.** Process for the preparation of a reagent for the sample preparation for the determination of vitamin B12 in blood serum, characterised in that 1 to 20 mg/ml of liponic acid, 1 to 10 mg/ml of an alkali metal cyanide at pH 10 to 14 are used as active component materials.

## Revendications

**Revendications pour les Etats contractants suivants : AT, BE, CH, LI, DE, DK, FR, GB, GR, IT, LU, NL, SE**

**1.** Procédé pour séparer un analyte lié à une protéine de liaison par dégradation de la protéine de liaison par un thiol clivant les groupes SH dans le domaine alcalin, caractérisé en ce que l'on utilise comme thiol clivant les groupes SH un acide 1,2-dithiolane-3-carboxylique de formule (I)

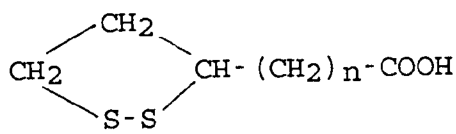

(I)

dans laquelle n représente un nombre entier de 1 à 8.

**2.** Procédé selon la revendication 1, caractérisé en ce que, dans la formule (I), n représente 3 à 5.

**3.** Procédé selon la revendication 1 ou 2, caractérisé en ce que l'on utilise l'acide lipoïque comme acide de formule (I).

**4.** Procédé selon l'une des revendications 1 à 3, caractérisé en ce que la concentration de l'acide 1,2-dithiolane-3-carboxylique de formule (I) (calculée avec n = 4 pour l'acide lipoïque) est de 1 à 20 mg/ml.

**5.** Procédé selon l'une des revendications 1 à 4, caractérisé en ce que l'analyte qui doit être libéré est une vitamine, une hormone thyroïdienne ou un steroïde.

**6.** Utilisation du procédé selon l'une des revendications 1 à 5 pour la préparation d'echantillon pour des procédés d'analyse et d'examen pour la détermination de l'analyte libéré, en particulier pour des immunoanalyses.

**7.** Utilisation d'un acide 1,2-dithiolane-3-carboxylique de formule (I)

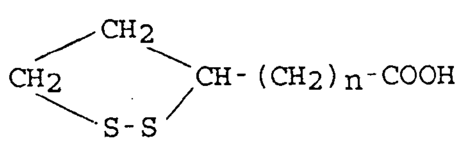

(I)

dans laquelle n représente un nombre entier de 1 à 8, de préférence de 3 à 5, et en particulier 4, pour séparer un analyte lié à une protéine de liaison comme préparation d'echantillon pour sa détermination, en particulier par immunoanalyse.

**8.** Réactif pour la préparation d'échantillon pour la détermination de la vitamine B12 dans le sérum sanguin, contenant

1 à 20 mg/ml d'acide lipoïque,
1 à 10 mg/ml de cyanure alcalin

à pH 10 à 14.

**Revendications pour l'Etat contractant suivant : ES**

1. Procédé pour séparer un analyte lié à une protéine de liaison par dégradation de la protéine de liaison par un thiol clivant les groupes SH dans le domaine alcalin, caractérisé en ce que l'on utilise comme thiol clivant les groupes SH un acide 1,2-dithiolane-3-carboxylique de formule (I)

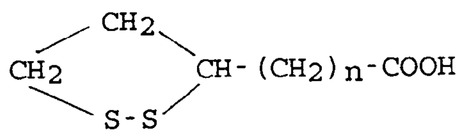

(I)

dans laquelle n représente un nombre entier de 1 à 8.

2. Procédé selon la revendication 1, caractérisé en ce que, dans la formule (I), n représente 3 à 5.

3. Procédé selon la revendication 1 ou 2, caractérisé en ce que l'on utilise l'acide lipoique comme acide de formule (I).

4. Procédé selon l'une des revendications 1 à 3, caractérisé en ce que la concentration de l'acide 1,2-dithiolane-3-carboxylique de formule (I) (calculée avec n = 4 pour l'acide lipoïque) est de 1 à 20 mg/ml.

5. Procédé selon l'une des revendications 1 à 4, caractérisé en ce que l'analyte qui doit être libéré est une vitamine, une hormone thyroïdienne ou un stéroïde.

6. Utilisation du procédé selon l'une des revendications 1 à 5 pour la préparation d'echantillon pour des procédés d'analyse et d'examen pour la détermination de l'analyte libéré, en particulier pour des immunoanalyses.

7. Utilisation d'un acide 1,2-dithiolane-3-carboxylique de formule (I)

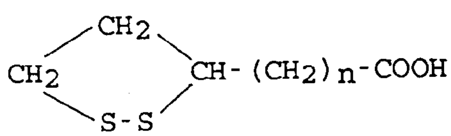

(I)

dans laquelle n représente un nombre entier de 1 à 8, de préférence de 3 à 5, et en particulier 4, pour séparer un analyte lié à une protéine de liaison comme préparation d'échantillon pour sa détermination, en particulier par immunoanalyse.

8. Procédé de préparation d'un réactif pour la préparation d'échantillon pour la détermination de la vitamine B12 dans le sérum sanguin, caractérisé en ce que l'on utilise comme constituants actifs

   1 à 20 mg/ml d'acide lipoïque,

   1 à 10 mg/ml de cyanure alcalin

   à pH 10 à 14.

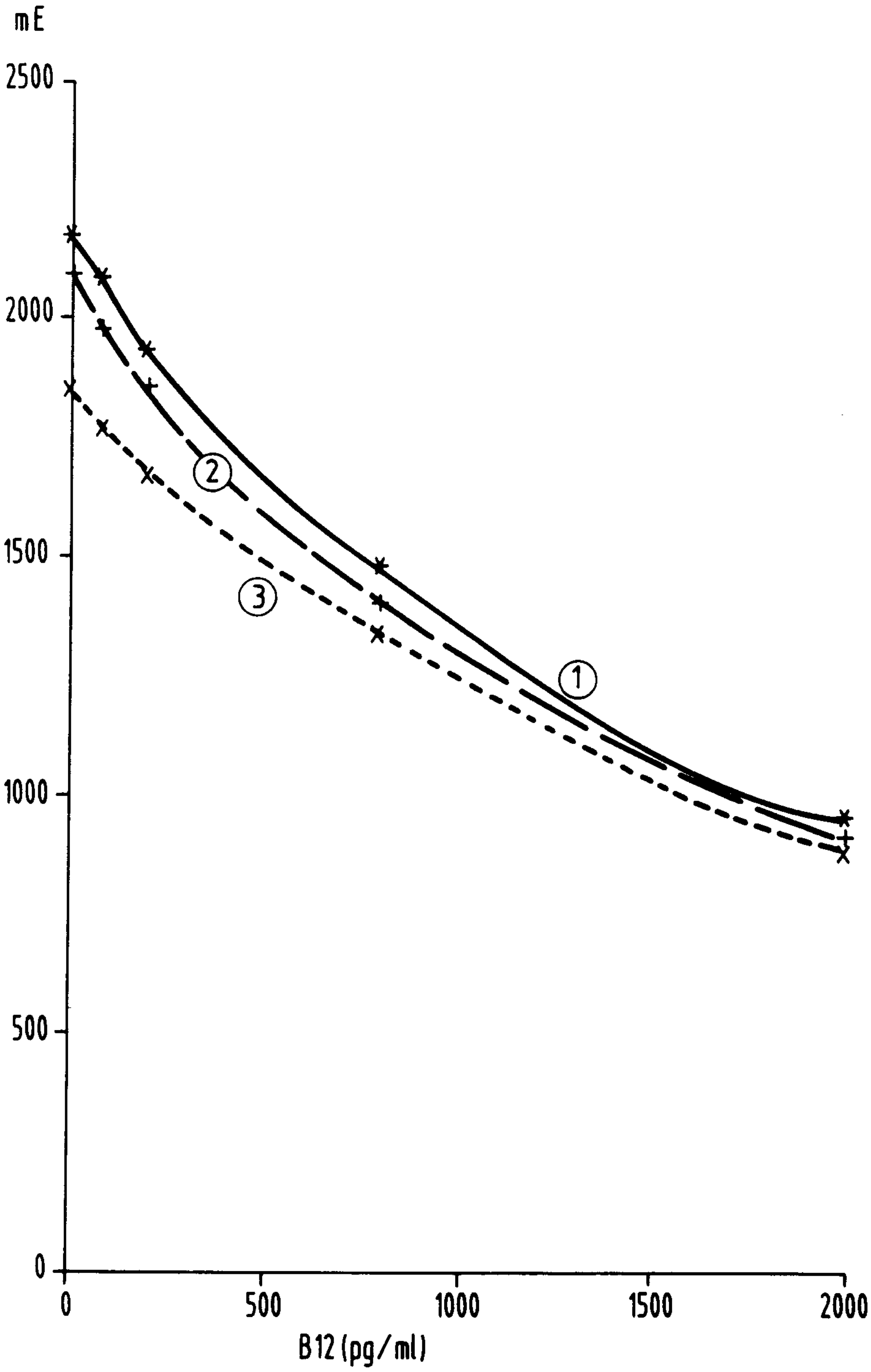
mE
2500
2000
1500
1000
500
0
0
500
1000
1500
2000
B12 (pg/ml)
1
2
3